# EUROPEAN PATENT SPECIFICATION

(11) **EP 4 355 234 B1**
(45) Date of publication and mention of the grant of the patent: **19.08.2026**
(21) Application number: 22738257.9
(22) Date of filing: 15.06.2022
(51) Int. Cl.: A61B 17/86

(54) **SELF-PUNCHING BONE ANCHOR**
SELBSTSTANZENDEN KNOCHENANKER
ANCRAGE OSSEUX AUTO-PERFORANT

(30) Priority: 15.06.2021 US 202163210733 P
(43) Date of publication of application: 24.04.2024
(73) Proprietor: CONMED Corporation, Largo, FL 33773 (US)
(72) Inventor: THIBODEAU, Robert, Saint Petersburg, FL 33710 (US); BARTON, Patrick, Palm Harbor, FL 34685 (US)
(74) Representative: Strehl & Partner mbB
(86) International application number: PCT/US2022/033558
(87) International publication number: WO 2022/266183

(56) References cited:
- EP-A1- 1 749 490
- EP-A2- 3 841 988
- WO-A1-2012/121726
- WO-A1-2017/164876
- US-A1- 2019 343 507

## Description

### 1. TECHNICAL FIELD OF THE INVENTION

The present disclosure relates to orthopedic bone anchors and, more particularly, to a bone anchor tip for penetrating hard or dense bone.

### 2. DESCRIPTION OF THE RELATED ART

Self-Punching bone anchors are commonly used in arthroscopic repairs because a pre-punched pilot hole is not required for implantation. This approach lets the clinician avoid the potential hardship and hassle of re-locating the pre-punched osteotomy in order to insert the anchor, which can be difficult if there is residual soft tissue at the implantation site. Self-punching also removes the implantation error of not implanting the bone anchor along the same longitudinal axis as the pilot hole, which can lead to misalignment and bone anchor fracture. Most conventional self-punching bone anchors use a conical or conical-like tip for creating the osteotomy in the bone. These conical tips are effective in soft to medium density bone, but struggle to penetrate in harder, denser bone.

An anchor from which the pre-characterizing part of claim 1 starts out is disclosed in WO 2012/121726 A1. Related art is disclosed in US 2019/343507 A1. Further related art is disclosed in the document EP 3 841 988 A2.

### BRIEF SUMMARY

Accordingly, there is a need for an improved self-punching bone anchor tip that can penetrate harder, denser bone. In response to this need, the invention proposes a anchor for insertion into a bone according to claim 1. The anchor disclosed herein is a self-punching bone anchor and tip that provides a more efficient cutting geometry than allows for penetration into harder and denser bone. The cutting geometry of the bone anchor also greatly reduces the frequency of skiving off the bone surface during implantation due to the unique cutting geometry. Osteotomies created by the anchor disclosed herein are thus created more efficiently, with less mallet strikes than osteotomies created with a conventional conical or conical-like tip and are much less likely to cause the bone to crack during osteotomy formation. The efficiency of the cutting geometry of the anchor disclosed herein also allows for softer materials such as polymers, biocomposite, and biologic materials to be used as a base material for the punching tips and anchors, thereby opening up many more possibilities for regenerative care without the need for metal components to be left behind in the body.

According to one aspect of the design, a anchor for insertion into bone has a body extending along a longitudinal axis and at least two cutting edges extending along the body and converging at an end of the body to form a point in alignment with the longitudinal axis. The cutting edges define a cutting face therebetween, which can be planar or curved. The cutting face may include a first portion extending at a first angle relative to the longitudinal axis and a second portion extending from the first portion at a second angle relative to the longitudinal axis that is different than the first angle. The body also includes a broach that has an outer surface that extends parallel to the longitudinal axis or that extends at an angle relative to the longitudinal axis. The broach may be at least partially interrupted by one of the at least one cutting faces, or fully interrupted by one of the at least one cutting faces. The body is intended to be coupled an anchor, such those that have a mechanical retention structure, are intended to change shape in response to insertion into a bone, or that have threads for advancing the anchor into a bone. The anchor and the body may be integrally formed together, and the anchor and the body may be formed from different materials.

### BRIEF DESCRIPTION OF THE SEVERAL VIEWS OF THE DRAWING(S)

The present invention will be more fully understood and appreciated by reading the following Detailed Description in conjunction with the accompanying drawings, in which:
FIG. 1 is a schematic of a self-punching tip for a bone anchor .
FIG. 2 is a side view of a self-punching tip for a bone anchor having three cutting edges defining three cutting surfaces.
FIG. 3 is an end view of a self-punching tip for a bone anchor having three cutting edges defining three cutting surfaces.
FIG. 4 is a side view of a self-punching tip for a bone anchor having four cutting edges defining four cutting surfaces.
FIG. 5 is an end view of a self-punching tip for a bone anchor having four cutting edges defining four cutting surfaces
FIG. 6 is a side view of a self-punching tip for a bone anchor having six cutting edges defining six cutting surfaces
FIG. 7 is an end view of a self-punching tip for a bone anchor having six cutting edges defining six cutting surfaces.
FIG. 8 is a perspective view of a self-punching tip for a bone anchor having flat cutting faces.
FIG. 9 is an end view of a self-punching tip for a bone anchor having flat cutting faces.
FIG. 10 is a perspective view of a self-punching tip for a bone anchor having curved cutting faces.
FIG. 11 is an end view of a self-punching tip for a bone anchor having curved cutting faces.
FIG. 12 is a side view of a self-punching tip for a bone anchor having a single angle cutting face.
FIG. 13 is a side view of a self-punching tip for a bone anchor having a multiangle cutting face.
FIG. 14 is a side view of a self-punching tip for a bone anchor having dual interrupted broches.
FIG. 15 is a side view of a self-punching tip for a bone anchor having dual tiered broches.
FIG. 16 is a side view of a self-punching tip for a bone anchor having multiple interrupted broches.
FIG. 17 is a side view of a self-punching tip for a bone anchor having dual tiered broches that extend parallel to the longitudinal axis of the self-punching tip.
FIG. 18 is a side view of a self-punching tip for a bone anchor having dual tiered broches that extend obliquely to the longitudinal axis of the self-punching tip.
FIG. 19 is a side view of a self-punching tip for a bone anchor having fully interrupted broaches.
FIG. 20 is a side view of a self-punching tip for a bone anchor having partially interrupted broaches.
FIG. 21 is a side view of a self-punching tip for a bone anchor having uninterrupted broaches
FIG. 22 is a series of side view of different anchor bodies that may be outfitted with a self-punching tip.
FIG. 23 is a pair of side view of single and multi-body anchors for use with a self-punching tip.
FIG. 24 is a series of side view of different anchor body compositions for use with a self-punching tip.

### DETAILED DESCRIPTION

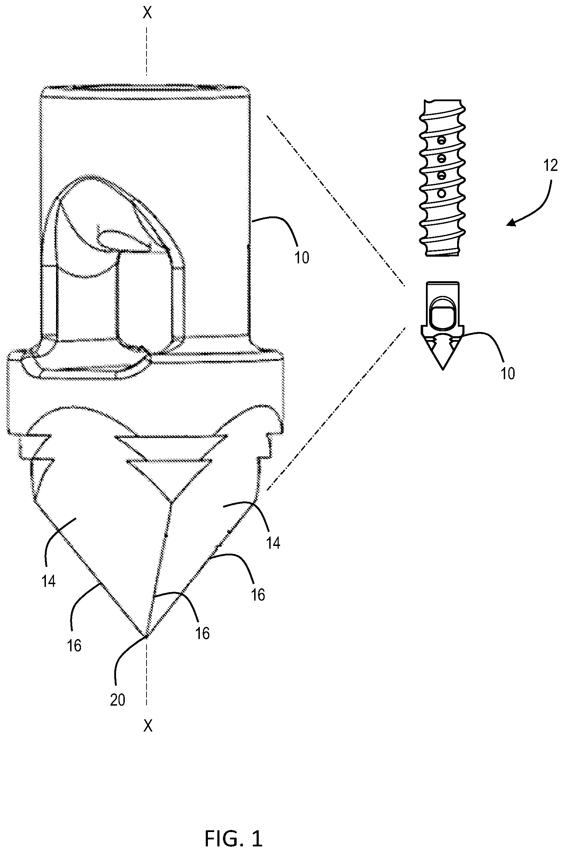

Referring to the figures, wherein like numerals refer to like parts throughout, there is seen in FIG. 1 a self-punching tip 10 for use as part of a bone anchor 12 having a geometry that is designed to increase the self-punching efficiency of anchor 12 during installation and thus change the manner in which osteotomies are formed. Tip 10 can initiate and propagate osteotomies by cutting into and then broaching the bone material during insertion as compared to traditional conical punching tips that simply pierce and then dilate the bone material. Tip 10 includes a series of cutting faces 14 that are flat or curved and extend at an angle to the longitudinal axis X-X of tip 10 and terminate in a point 20 in alignment with longitudinal axis X-X. Cutting faces 14 are defined by a plurality of cutting edges 16 that intersect at the point 20 of tip 10. The angle of cutting faces 14 is preferably as small as possible, however, the overall length of tip 10 should not exceed the minimum depth of a conventional pilot hole. The diameter of tip 10 is governed by the diameter of anchor 12 to which tip 10 is attached and may be any conventional diameter in use for bone installations. For harder materials, or if a user required less installation force, a more sharply angled tip 10 may be used. For softer materials, a less angled tip 10 may suffice. An angle for each cutting face of 22 degrees relative to the longitudinal axis, resulting in an inclusive angle of 44 degrees for tip 10 as a whole, has been found effective. It should be recognized that this angle can be varied for the particular bone or surgical operation for which anchor 12 is intended.

During insertion, tip 10 maintains multiple points of contact with the bone, including one at point 20 and one for each cutting edge 16, for the duration of the insertion. The multiple locations of contact significantly reduce the active surface area as compared to conventional conical tips and improve the cutting efficiency of tip 10 by greatly reducing the drag of friction, minimizing the surfaces area in which the opposing forces of the bone material can act on anchor 12, and interrupting the hoop stress compression of the bone seen during dilation by cutting into the bone in multiple locations. Tip 10 thus assists when inserting an anchor into bone when provided on an anchor. Tip 10 (and anchor) are thus positioned proximately to a location of a bone and a force applied along the longitudinal axis of the anchor until tip 10 forms a hole in the bone and the anchor can be fully inserted into the hole.

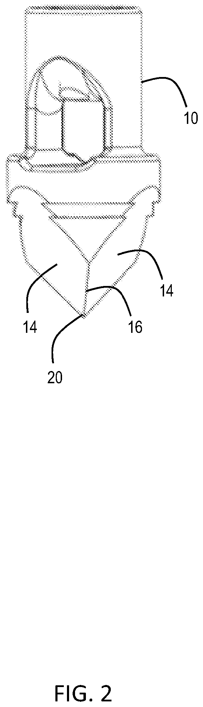

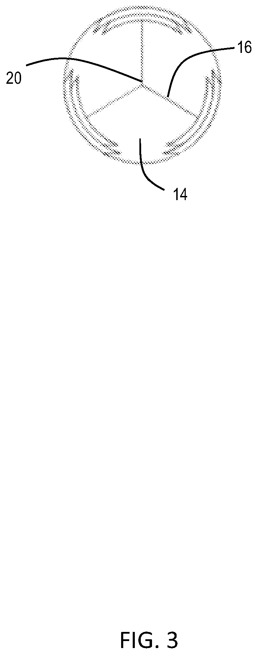

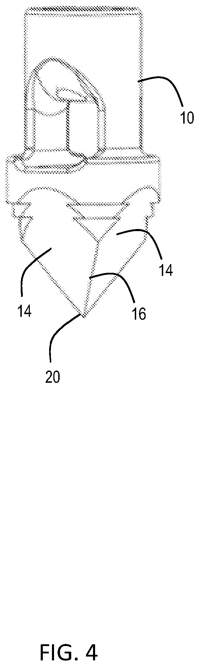

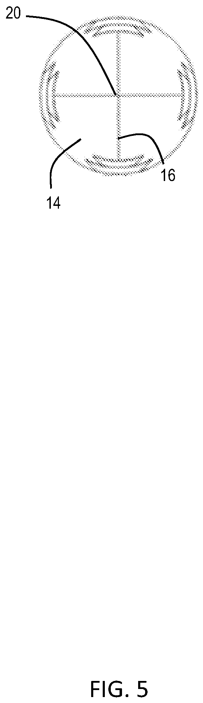

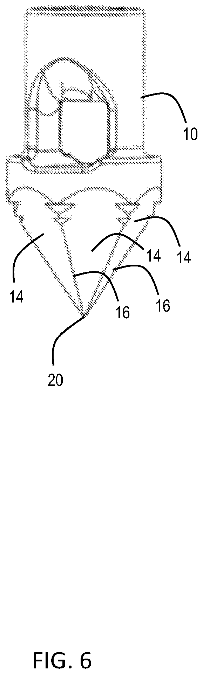

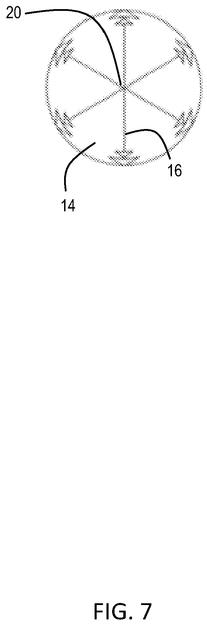

Tip 10 can have any number of cutting faces 14, as seen in the various embodiments of FIGS. 2-7. More specifically, as seen in FIGS. 2 and 3, tip 10 may have three faces 14 defined by three cutting edges 16. As seen in FIGS. 4 and 5, tip 10 may four faces 14. As seen in FIG. 6 and 7, tip 10 may have six faces 14. Generally, the more faces 14 that can be employed, the more easily tip can form a pilot hole during installation into a bone.

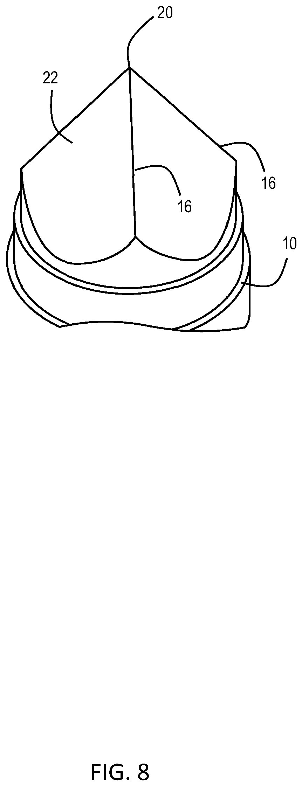

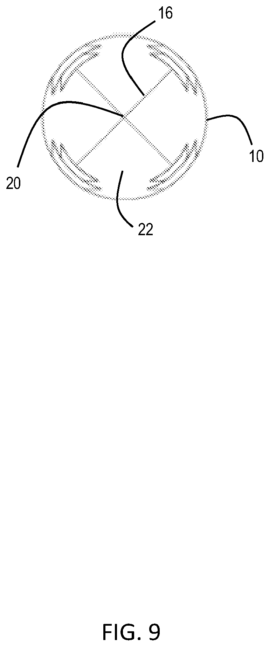

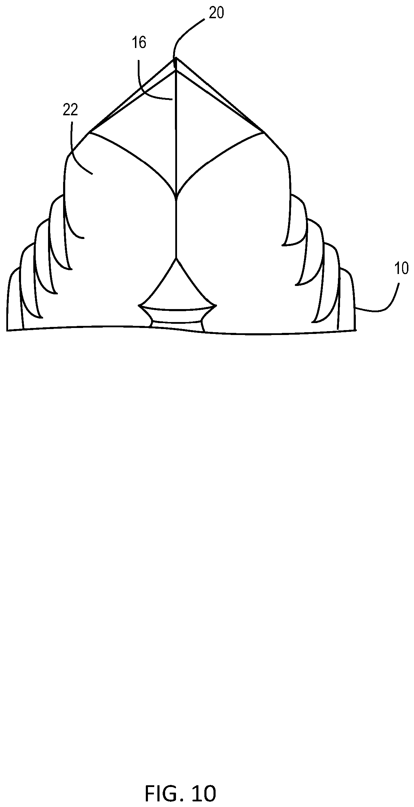

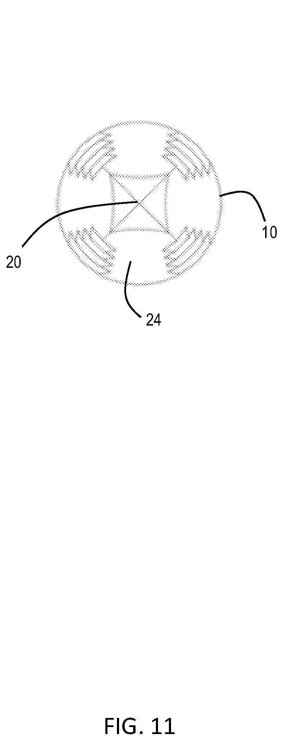

Referring to FIGS. 8 and 9, cutting faces 14 may be flat 22, i.e., cutting faces 14 may extend along plane that intersects axis X-X. Referring to FIGS. 10 and 11, cutting faces 14 may be also be curved 24 by extending in a concave or convex fashion, or any combination thereof.

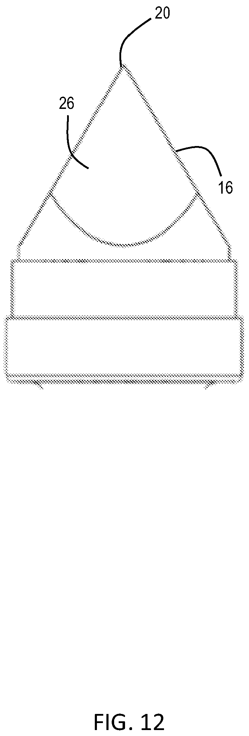

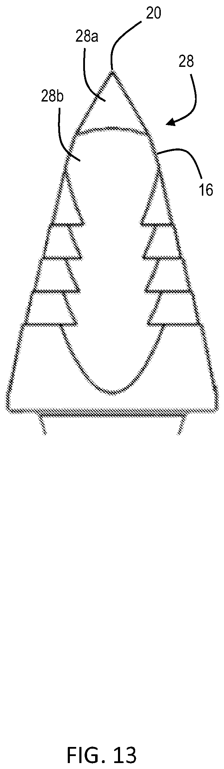

Referring to FIGS. 12 and 13, cutting edges 16 can have single face 26, as seen in FIG. 12, or a multi-angle face 28 defined by at least a first section 28a that extends at an angle to at least a second section 28b, as seen in FIG. 13.

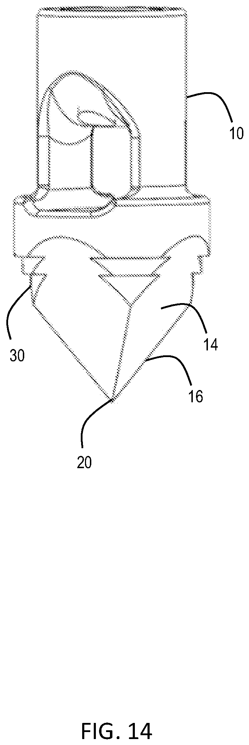

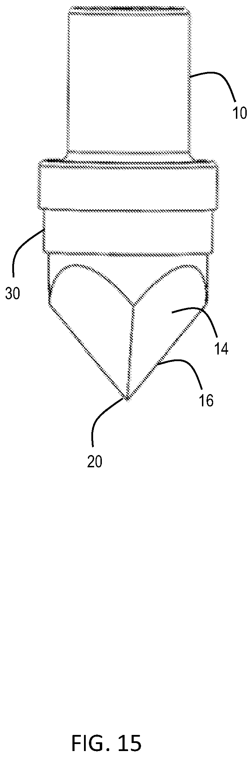

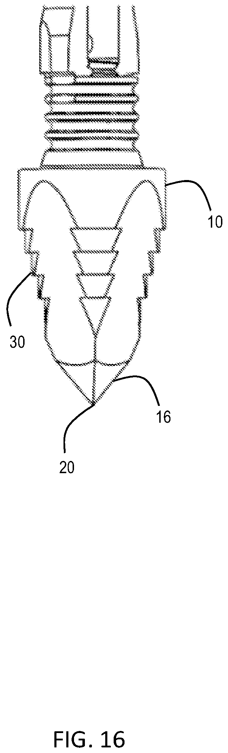

Referring to FIGS. 14 through 16, tip 10 includes a plurality of broaches 30 positioned circumferentially around at least a part of tip 10. Broaches 30 assist with the removal of material during insertion as is understood in the machining art, as opposed to simply pushing bone to the sides as is the case with conventional conical design self-punching anchors. Broaches 30 may be present in various frequency, have various dimensions, and positioning relative to each other. For example, as seen in FIG. 14, tip 10 may have two tightly positioned broaches 30. As seen in FIG. 15, tip 10 may have two broaches that are spaced out. As seen in FIG. 16, tip 10 may also have several tightly positioned broaches 30, shown in FIG. 16 as four in total. The number and positioning of broaches 30 can be varied for use with different hardness bone and to accommodate different impact force requirements. Broaches 30 provide for a more precise diameter hole as the hole will not expand after removal of tip 10 as is the case with conical tip anchors, and can allow for the easier removal of tip 10 or anchor 12 than is the case with conical tip anchors. As should be recognized from the illustrated broaches 30, the hole formed by tip 10 does not need to be circular, and thus offers geometric shapes not possible with a conical anchor tip.

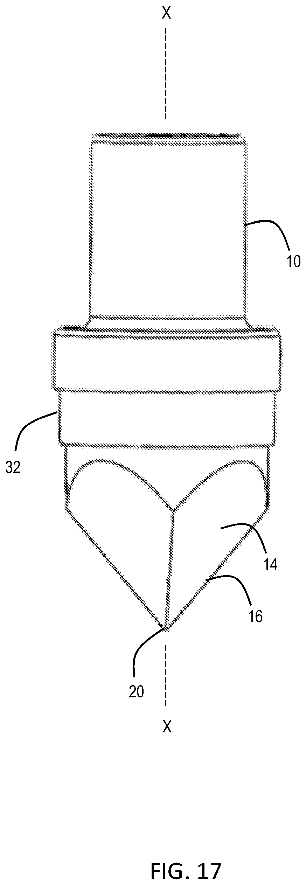

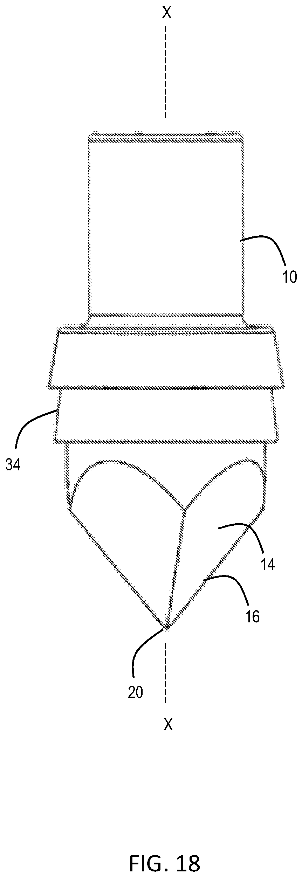

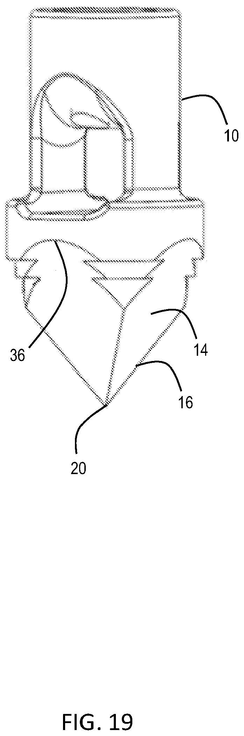

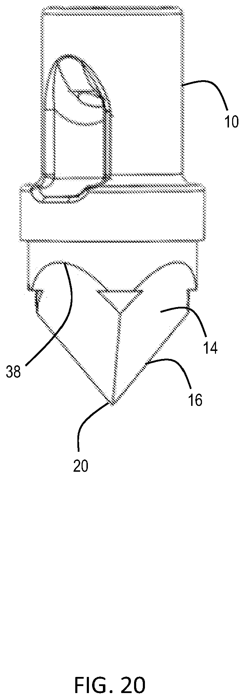

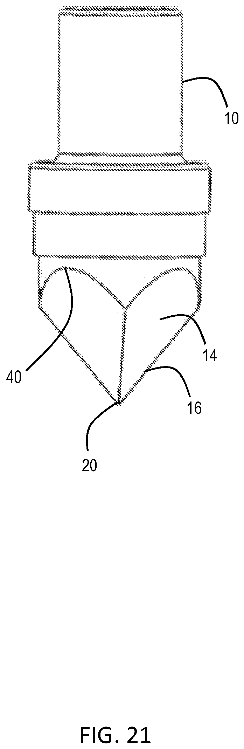

Referring to FIG. 17, broaches 30 can extend parallel 32 to the longitudinal axis X-X of tip 10, as seen in FIG. 17, or extend at an angle 34 that is oblique relative to the longitudinal axis, as seen in FIG. 18. Broaches 30 can also be interrupted 36 as seen in FIG. 19, partially interrupted 38 as seen in FIG. 20, non-interrupted 40 as seen in FIG. 21, or any combination therein. Interruptions of broaches 30 can help stabilize tip 10 against undesirable rotation during installation.

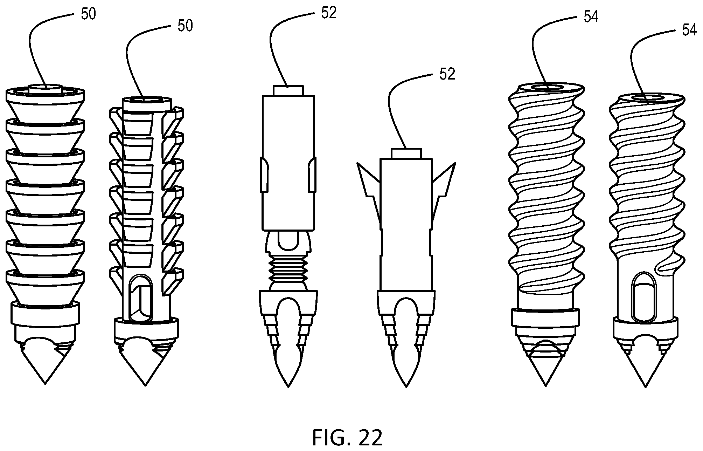

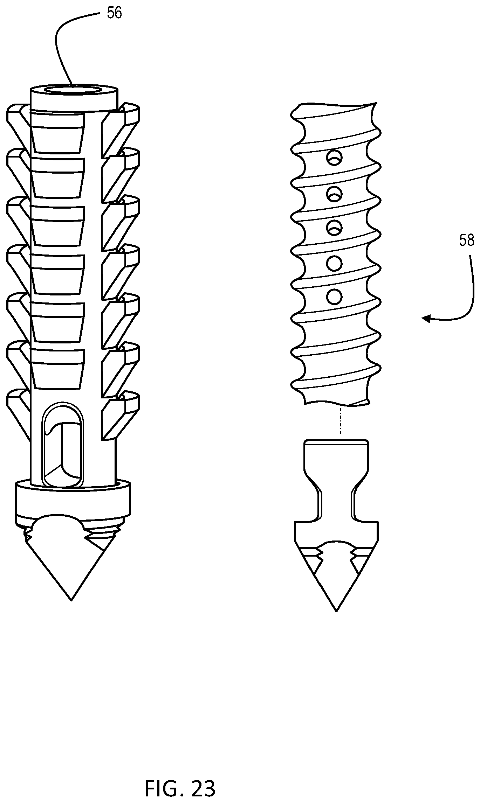

Tip 10 may be used in connection with different types of bone anchors 12, including conventional anchors 12 such as those having built-in mechanical retention features 50, changeable mechanical retention features 52 achieved through a shape or conformational change, screw-type anchors 12 that can be screwed into the bone 54, as seen in FIG. 22. Tip 10 can also be incorporated into anchor 12 having a single body 56 or as a separate component of anchor 12 formed as a multi-body anchor 58, as seen in FIG. 23. Tip 10 may also be used in any joint space or surgical location where self-punching anchors can be or are currently utilized.

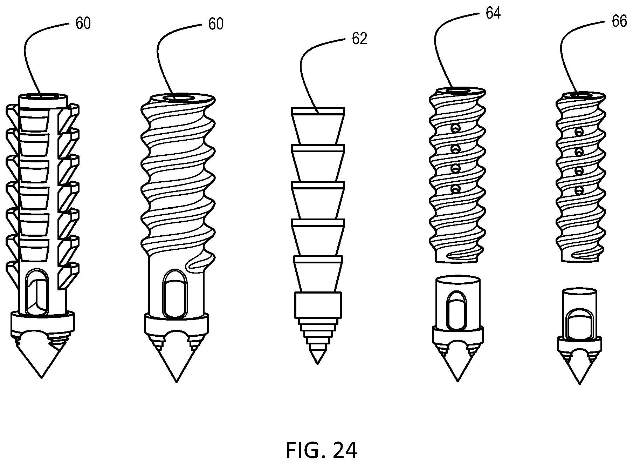

Tip 10 can be used with traditional biocompatible materials currently used for making osteotomies such as biocompatible metals like stainless steel, titanium, and titanium alloys. Due to the increased cutting efficiency of tip 10, other softer biocompatible materials such as PEEK, biocomposite materials, and bioglasses can be used. Tip 10 may also be used on anchors 12 having a single body 60 comprising the same material as tip 10, anchors 12 having a single body 62 comprised of multiple materials, anchors 12 having a multibody comprised of the same material 64, and anchors 12 having a multibody comprised of multiple materials 66, as seen in FIG. 24.

## Claims

1. An anchor for insertion into a bone, comprising:
a body;
a tip (10) coupled to the body and extending along a longitudinal axis (X-X); and
at least two cutting edges (16) extending along the tip and defining at least one cutting face (14) therebetween, wherein the at least two cutting edges (16) converge at an end of the tip to form a point (20) in alignment with the longitudinal axis,
**characterized in that**
the tip includes at least one broach (30).

2. The anchor of claim 1, wherein the body includes at least one mechanical feature (50~54) for engaging with a bone into which the anchor is inserted.

3. The anchor of claim 2, wherein the mechanical feature (52) comprises a structure that changes shape in response to insertion of the anchor into a bone.

4. The anchor of claim 1, wherein the tip and (10) the body (56) are integrally formed.

5. The anchor of claim 1, wherein the tip (10) is formed from a first material and the body (66) is formed from a second material that is different than the first material.

6. The anchor of claim 1, wherein the at least one cutting face (14) is planar.

7. The anchor of claim 1, wherein the at least one cutting face (14) is curved.

8. The anchor of claim 1, wherein the at least one cutting face (14) includes a first portion (28a) extending at a first angle relative to the longitudinal axis and a second portion (28b) extending from the first portion at a second angle relative to the longitudinal axis that is different than the first angle.

9. The anchor of claim 1, wherein the at least one broach (30) has an outer surface that extends parallel to the longitudinal axis.

10. The anchor of claim 9, wherein the at least one broach has an outer surface that extends at an angle relative to the longitudinal axis.

11. The anchor of claim 9, wherein the at least one broach (30) is at least partially interrupted by one of the at least one cutting face (14).

12. The anchor of claim 9, wherein the at least one broach (30) is fully interrupted by one of the at least one cutting face (14).

## Patentansprüche

1. Anker zum Einsetzen in einen Knochen, umfassend:
einen Körper;
eine Spitze (10), die mit dem Körper gekoppelt ist und sich entlang einer Längsachse (X-X) erstreckt; und
mindestens zwei Schneidkanten (16), die sich entlang der Spitze erstrecken und mindestens eine Schneidfläche (14) zwischen einander definieren, wobei die mindestens zwei Schneidkanten (16) an einem Ende der Spitze zusammenlaufen, um einen Punkt (20) in Ausrichtung mit der Längsachse zu bilden,
**dadurch gekennzeichnet, dass**
die Spitze mindestens einen Räumer (30) umfasst.

2. Anker nach Anspruch 1, wobei der Körper mindestens ein mechanisches Merkmal (50 - 54) zum Eingriff mit einem Knochen umfasst, in den der Anker eingesetzt wird.

3. Anker nach Anspruch 2, wobei das mechanische Merkmal (52) eine Struktur umfasst, die ihre Form als Reaktion auf das Einsetzen des Ankers in einen Knochen ändert.

4. Anker nach Anspruch 1, wobei die Spitze (10) und der Körper (56) einstückig gebildet sind.

5. Anker nach Anspruch 1, wobei die Spitze (10) aus einem ersten Material gebildet ist und der Körper (66) aus einem zweiten Material gebildet ist, das sich von dem ersten Material unterscheidet.

6. Anker nach Anspruch 1, wobei die mindestens eine Schneidfläche (14) flach ist.

7. Anker nach Anspruch 1, wobei die mindestens eine Schneidfläche (14) gekrümmt ist.

8. Anker nach Anspruch 1, wobei die mindestens eine Schneidfläche (14) einen ersten Abschnitt (28a) umfasst, der sich in einem ersten Winkel relativ zur Längsachse erstreckt, und einen zweiten Abschnitt (28b), der sich von dem ersten Abschnitt in einem zweiten Winkel relativ zur Längsachse erstreckt, der sich von dem ersten Winkel unterscheidet.

9. Anker nach Anspruch 1, wobei der mindestens eine Räumer (30) eine Außenfläche aufweist, die sich parallel zur Längsachse erstreckt.

10. Anker nach Anspruch 9, wobei der mindestens eine Räumer eine Außenfläche aufweist, die sich in einem Winkel relativ zur Längsachse erstreckt.

11. Anker nach Anspruch 9, wobei der mindestens eine Räumer (30) zumindest teilweise durch eine der mindestens einen Schneidfläche (14) unterbrochen ist.

12. Anker nach Anspruch 9, wobei der mindestens eine Räumer (30) vollständig durch eine der mindestens einen Schneidfläche (14) unterbrochen ist.

## Revendications

1. Ancrage pour insertion dans un os, comprenant :
un corps ;
une pointe (10) couplée au corps et s'étendant le long d'un axe longitudinal (X-X) ; et
au moins deux bords de coupe (16) s'étendant le long de la pointe et définissant au moins une face de coupe (14) entre ceux-ci, dans lequel les au moins deux bords de coupe (16) convergent à une extrémité de la pointe pour former un point (20) en alignement avec l'axe longitudinal,
**caractérisé en ce que**
la pointe inclut au moins une broche (30).

2. Ancrage selon la revendication 1, dans lequel le corps inclut au moins une caractéristique mécanique (50~54) pour engagement avec un os dans lequel l'ancrage est inséré.

3. Ancrage selon la revendication 2, dans lequel la caractéristique mécanique (52) comprend une structure qui change de forme en réponse à l'insertion de l'ancrage dans un os.

4. Ancrage selon la revendication 1, dans lequel la pointe (10) et le corps (56) sont formés en une seule pièce.

5. Ancrage selon la revendication 1, dans lequel la pointe (10) est constituée d'un premier matériau et le corps (66) est constitué d'un deuxième matériau qui est différent du premier matériau.

6. Ancrage selon la revendication 1, dans lequel l'au moins une face de coupe (14) est plane.

7. Ancrage selon la revendication 1, dans lequel l'au moins une face de coupe (14) est incurvée.

8. Ancrage selon la revendication 1, dans lequel l'au moins une face de coupe (14) inclut une première partie (28a) s'étendant selon un premier angle par rapport à l'axe longitudinal et une deuxième partie (28b) s'étendant depuis la première partie à un deuxième angle par rapport à l'axe longitudinal qui est différent du premier angle.

9. Ancrage selon la revendication 1, dans lequel l'au moins une broche (30) a une surface extérieure qui s'étend parallèle à l'axe longitudinal.

10. Ancrage selon la revendication 9, dans lequel l'au moins une broche a une surface extérieure qui s'étend selon un angle par rapport à l'axe longitudinal.

11. Ancrage selon la revendication 9, dans lequel l'au moins une broche (30) est au moins partiellement interrompue par une de l'au moins une face de coupe (14).

12. Ancrage selon la revendication 9, dans lequel l'au moins une broche (30) est totalement interrompue par une de l'au moins une face de coupe (14).
